# EUROPEAN PATENT APPLICATION

(11) **EP 3 278 806 A1**
(43) Date of publication of application: **07.02.2018**
(21) Application number: 16773215.5
(22) Date of filing: 01.04.2016
(51) Int. Cl.: A61K 31/7008, A61P 3/10, A61P 25/00, A61P 43/00

(54) **GLUCOSE-SENSITIVE CELL PROTECTING AGENT**

(30) Priority: 03.04.2015 JP 2015077228
(71) Applicant: The University of Tokyo, Tokyo 113-8654 (JP)
(72) Inventor: SHIOTA, Kunio, Tokyo 113-8654 (JP); HAYAKAWA, Koji, Tokyo 113-8654 (JP); TAKAMORI, Mitsuko, Tokyo 113-8654 (JP); FUJII, Ryosuke, Tokyo 113-8654 (JP); ITO, Yukishige, Wako-shi Saitama 351-0198 (JP)
(74) Representative: J A Kemp
(86) International application number: PCT/JP2016/060882
(87) International publication number: WO 2016/159335

(57) **Abstract**

Provided is a means for reducing the adverse effects of high glucose concentration on the living body.

An agent containing N-acetyl-D-mannosamine for protecting a glucose-sensitive cell from glucose toxicity due to high glucose concentration; a pharmaceutical composition for protecting a glucose-sensitive cell from glucose toxicity due to high glucose concentration, containing an effective amount of N-acetyl-D-mannosamine and a pharmaceutically acceptable carrier; use of N-acetyl-D-mannosamine in producing a medicament for protecting a glucose-sensitive cell from glucose toxicity due to high glucose concentration; and a method of protecting a glucose-sensitive cell from glucose toxicity due to high glucose concentration, including administering an effective amount of N-acetyl-D-mannosamine to a subject in need thereof. The glucose-sensitive cell is selected from the group consisting of a nerve cell, a renal cell and a vascular endothelial cell.

## Description

### [Technical Field]

The present invention relates to a protective agent for glucose-sensitive cells, and more specifically relates to a protective use of N-acetyl-D-mannosamine against adverse effects of high glucose concentration on the glucose-sensitive cells such as nerve cells and the like.

### [Background Art]

As diabetic complications, diabetic retinopathy, diabetic nephropathy, vascular complication, skin complication, immunodeficiency, neuropathy, hypertension and the like are known. Since the mechanism by which hyperglycemia causes these complications is not clear, an effective therapeutic drug does not exist. Furthermore, it has been reported that chronic hyperglycemia not only causes these diseases, but may also become neurotoxicity to the central nervous system (glucose neurotoxicity) (non-patent documents 1-4).

N-acetyl-D-mannosamine, which is an isomer of N-acetyl-D-glucosamine, is known as, for example, a starting material for the enzymatic synthesis of sialic acid (N-acetylneuraminic acid) to be a pharmaceutical product or a starting material for a medicament. In addition, N-acetyl-D-mannosamine permits enzymatic synthesis of a sialic acid derivative from derivatives thereof, hence an industrially important substance. As a production method of N-acetyl-D-mannosamine, a method including increasing the molar conversion yield into N-acetylmannosamine by adding boric acid or borate during isomerization of N-acetylglucosamine under alkaline conditions is known (patent document 1). In addition, a method of producing N-acetyl-D-mannosamine by reacting N-acetylneuraminic acid lyase with sialic acid as a substrate is also known (patent document 2). A method of regulating lectin binding to a cellular surface and a method of regulating proliferation of nerve cells, each by contacting an acylated form of N-mannosamine with the cells, have been proposed (patent document 3). In patent document 3, N-acetyl-D-mannosamine is considered a negative control in promoting the axon growth in vitro. Furthermore, mutation (e.g., M712T mutation resulting from point mutation) in GNE gene encoding uridine diphbspho-N-acetylglucosamine 2-epimerase/N-acetylmannosamine (ManNAc) kinase (GNE/MNK), which is the main enzyme of sialic acid biosynthesis, is known to cause autosomal recessive hereditary inclusion body myopathy (HIBM) which is developed in adults. It has been reported that Gne/Mnk M712T homozygous mutant mouse did not survive beyond age 3 after birth, and developed serious glomerular proteinuria; however, administration of N-acetylmannosamine (ManNAc) rescued the mice (non-patent document 5).

The present inventors have found that N-acetyl-D-mannosamine is effective for the improvement of functional deterioration of brain and improvement of sleep disorders (patent documents 4 and 5). In addition, the present inventors have succeeded in efficiently producing orexin neurons from pluripotent stem cells or neural progenitor cells by using N-acetyl-D-mannosamine (patent document 6). Furthermore, the present inventors have found that N-acetyl-D-mannosamine is useful for the treatment of depression (patent document 7). It is known that depression increases the onset of diabetes and the mortality due to diabetes, progresses arteriosclerosis, increases the risk of myocardial infarction and cerebral infarction, and also increases mortality due to cancer; however, a physiological or pathological relationship between depression and the progression of these diseases is unknown.

### [Document List]

### [Patent documents]

patent document 1: JP-A-hei10-182685
patent document 2: JP-A-2001-78794
patent document 3: US Patent No. 6274568
patent document 4: WO 2010/027028
patent document 5: JP-A-2011-178702
patent document 6: WO 2013/047773
patent document 7: WO 2015/174532

### [non-patent document]

non-patent document 1: Nature Reviews Neuroscience, vol. 9, pp.36-45, 2008
non-patent document 2: Nature Reviews Neuroscience, vol. 15, pp.367-378, 2014
non-patent document 3: Nature Reviews Endocrinology, vol. 7, pp.682-690, 2011
non-patent document 4: New England Journal of Medicine, 2013; 369:540-548
non-patent document 5: The Journal of Clinical Investigation, 2007; 117:1585-1594

### [SUMMARY OF THE INVENTION]

### [Problems to be Solved by the Invention]

Patients suffering from diabetes or pre-diabetic hyperglycemia are increasing on a worldwide scale, and it is required to prevent or treat diabetes and complications thereof at an early stage. An object of the present invention is to provide a means for reducing the adverse effects of high glucose concentration on the living body.

### [Means of Solving the Problems]

The present inventors have conducted intensive studies by using orexin nerve cells differentiated from human iPS cells, based on the induction method of orexin neurons disclosed in WO 2013/047773 and found that orexin nerve cells are glucose-sensitive and cannot be maintained under a high concentration glucose environment. In addition, they have unexpectedly found that disappearance of orexin nerve cells can be avoided by culturing in a medium containing N-acetyl-D-mannosamine, and N-acetyl-D-mannosamine has an action to protect nerves from neurotoxicity due to high glucose, which resulted in the completion of the present invention. The present inventors have also found that N-acetyl-D-mannosamine has an action to protect other glucose-sensitive cells from glucose toxicity, which resulted in the completion of the present invention.

Therefore, the present invention provides the following.
[1] An agent for protecting a glucose-sensitive cell from glucose toxicity due to high glucose concentration, comprising N-acetyl-D-mannosamine.
[2] The agent of [1], wherein the glucose-sensitive cell is selected from the group consisting of a nerve cell, a renal cell and a vascular endothelial cell.
[3] The agent of [1], wherein the glucose-sensitive cell is selected from the group consisting of a renal cell and a vascular endothelial cell, and protection of the glucose-sensitive cell refers to genome repair in the cell.
[4] A pharmaceutical composition for protecting a glucose-sensitive cell from glucose toxicity due to high glucose concentration, comprising an effective amount of N-acetyl-D-mannosamine and a pharmaceutically acceptable carrier.
[5] The pharmaceutical composition of [4], wherein the glucose-sensitive cell is selected from the group consisting of a nerve cell, a renal cell and a vascular endothelial cell.
[6] The pharmaceutical composition of [4], wherein the glucose-sensitive cell is selected from the group consisting of a renal cell and a vascular endothelial cell, and protection of the glucose-sensitive cell refers to genome repair in the cell.
[7] The pharmaceutical composition of any of [4] - [6], for the treatment of a diabetic complication.
[8] Use of N-acetyl-D-mannosamine in producing a medicament for protecting a glucose-sensitive cell from glucose toxicity due to high glucose concentration.
[9] The use of [8], wherein the glucose-sensitive cell is selected from the group consisting of a nerve cell, a renal cell and a vascular endothelial cell.
[10] The use of [8], wherein the glucose-sensitive cell is selected from the group consisting of a renal cell and a vascular endothelial cell, and protection of the glucose-sensitive cell refers to genome repair in the cell.
[11] A method of protecting a glucose-sensitive cell from glucose toxicity due to high glucose concentration, comprising administering an effective amount of N-acetyl-D-mannosamine to a subject in need thereof.
[12] The method of [11], wherein the glucose-sensitive cell is selected from the group consisting of a nerve cell, a renal cell and a vascular endothelial cell.
[13] The method of [11], wherein the glucose-sensitive cell is selected from the group consisting of a renal cell and a vascular endothelial cell, and protection of the glucose-sensitive cell refers to genome repair in the cell.
[14] The method of any of [11] - [13], for the treatment of a diabetic complication.
[15] An agent for protecting a nerve cell from disappearance due to high glucose concentration, comprising N-acetyl-D-mannosamine.
[16] The agent of [15], wherein the nerve cell is an orexin nerve cell.
[17] A pharmaceutical composition for the prophylaxis or treatment of nerve cell disappearance due to high glucose concentration, comprising an effective amount of N-acetyl-D-mannosamine and a pharmaceutically acceptable carrier.
[18] The pharmaceutical composition of [17], wherein the nerve cell is an orexin nerve cell.
[19] The pharmaceutical composition of [17] or [18], for the treatment of a diabetic complication.
[20] Use of N-acetyl-D-mannosamine in producing a medicament for protecting a nerve cell from disappearance due to high glucose concentration.
[21] The use of [20], wherein the nerve cell is an orexin nerve cell.
[22] A method for the prophylaxis or treatment of nerve cell disappearance due to high glucose concentration, comprising a step of administering an effective amount of N-acetyl-D-mannosamine to a subject in need thereof.
[23] The method of [22], wherein the nerve cell is an orexin nerve cell.
[24] The method of [22] or [23], for the prophylaxis or treatment of a diabetic complication.

### [Effect of the Invention]

N-acetyl-D-mannosamine is superior in the effect of protecting nerve cells exposed to high glucose concentration conditions or neural tissues in test subjects with hyperglycemia. Glucose-sensitive cells exposed to high glucose concentration conditions have lower resistance to genome DNA damage caused by various extracellular stresses. N-acetyl-D-mannosamine reduces glucose toxicity in the glucose-sensitive cells exposed to high glucose concentration conditions or test subjects with hyperglycemia. Specifically, it has an effect of imparting resistance to genome DNA damage or high repair capacity to the cells or tissues constituted of such cells. The therapeutic agent of the present invention containing N-acetyl-D-mannosamine as an active ingredient prevents cell death by protecting the functions of glucose-sensitive cells from hyperglycemia, and protects living organisms from hyperglycemia by recovering the decreased functions of cells due to hyperglycemia. Therefore, the therapeutic agent of the present invention is expected to be an anti-diabetes drug having a new working mechanism or used in combination with other anti-diabetes drugs and the like.

### [Brief Description of the Drawings]

Fig. 1 shows the effect of high glucose concentration on the nerve cells, by using orexin nerve cells differentiated from human iPS cells. In the Figure, IOC shows the orexin nerve cells obtained by culturing for 20 days in Reference Example 1. ManNAcF (or ManNFAc) is a fluoro derivative of ManNAc.
Fig. 2 shows the effect of hyperglycemia condition caused by ingestion of high-fat diet on the orexin nerve cells in the brain of diabetic model mice (db/db mouse). In the Figure, * shows t-test, and p<0.01 shows a significant difference.
Fig. 3 shows the effect of ManNAc and a derivative thereof on a decrease in the orexin production (glucose toxicity) when orexin nerve cells differentiated from human iPS cells were cultured for 8 days in the presence of high glucose.
Fig. 4 is a table which shows the effect of ManNAc and a derivative thereof when a differentiation induction system from human iPS cells into orexin nerve cells was used. It is clear that all derivatives show high induction activity into orexin cells, as compared to ManNAc.
Fig. 5 shows O-GlcNAc modification of histones when normal human renal proximal tubule epithelial cells (RPTEC) and RPTEC derived from Type II diabetes patients (D-RPTEC) were cultured under various glucose concentration (5 mM, 10 mM and 25 mM) conditions as investigated by an immunofluorescence staining method.
Fig. 6 shows the effect of ManNAc and derivatives thereof on O-GlcNAc modification of histones when renal proximal tubule epithelial cells derived from type II diabetes patients (D-RPTEC) were cultured under low glucose concentration (5 mM) or high glucose concentration (25 mM) condition as investigated by an immunofluorescence staining method. **; P<0.01.

### [Description of Embodiments]

The present invention provides an agent containing N-acetyl-D-mannosamine, for protecting glucose-sensitive cells from glucose toxicity due to high glucose concentration. In addition, the present invention provides an agent containing N-acetyl-D-mannosamine, for protecting nerve cells from disappearance due to high glucose concentration. The both agents are sometimes combinedly referred to as the agent of the present invention.

In the present invention, "glucose-sensitive cell" refer to a cell susceptible to the effects of extracellular glucose concentration, particularly high glucose concentration. Examples of the glucose-sensitive cell include a nerve cell, a renal cell, a vascular endothelial cell, a pancreatic islet β cell and the like, and the nerve cell, the renal cell and the vascular endothelial cell are preferable.

The "renal cell" is a cell constituting the kidney, and includes a glomerular endothelial cell, an epithelial cell of Bowman's capsule, a mesangial cell, a proximal renal tubule epithelial cell, a distal renal tubule epithelial cell and the like.

In the present invention, the "nerve cell" refers to a special cell for which cell body, axon and dendrite are considered as one unit and also called "neuron", which emits electrical signals, neurotransmitters or neuropeptides to carry biological regulating information. It may be any of nerve cells present in vivo, nerve cells taken out from in vivo to in vitro, nerve cells induced from stem cells in vitro, nerve cells maintained in vitro and transplanted in vivo, nerve cells in the brain, peripheral nerve cells, cultured cells derived from adult/fetus, and nerve cells produced artificially.

In the present invention, "protection of glucose-sensitive cell" means protection of a glucose-sensitive cell from glucose toxicity resulting from exposure to high glucose concentration conditions, or recovery of a glucose-sensitive cell from glucose toxicity. The glucose toxicity in the present specification means induction of genome DNA damage, decrease of genome repair capacity, cell disorder due to genome regulation abnormality, cell death, and the like, which are dependent on glucose concentration change. Protection of glucose-sensitive cells can be evaluated by using, as an index, the absence of significant decrees of genome repair in the cells under high glucose concentration conditions as compared to cells under low glucose concentration conditions. To be specific, it can be evaluated using modification of histones with O-type linked N-acetylglucosamine (O-GlcNAc) as an index. As used herein, the high glucose concentration refers to a glucose concentration in a medium or body fluid (preferably, blood) of not less than 7.1 mM (e.g., 25 mM), and the low glucose concentration refers to a glucose concentration in a medium or body fluid (preferably, blood) of less than 7.1 mM (e.g., 1 mM or 5 - 7 mM).

The present inventors have clarified that O-GlcNAc modification of histone varies by reacting with extracellular glucose (Filing No.: Application No. 2015-177176). O-GlcNAc modification is influenced by extracellular inflow of glucose and activation of hexosamine biosynthesis pathway associated therewith. The present inventors have found that, as shown in the below-mentioned Example 4, O-GlcNAc modification of histones increases under high glucose concentration conditions in normal renal proximal tubule epithelial cells (RPTEC), whereas it does not occur in RPTEC derived from type II diabetes patients (D-RPTEC), and that DNA repair capacity acquired by responding to glucose is not exhibited in the cells derived from diabetes patients. O-GlcNAc modification of histones increases in response to genome DNA damage, which indicates that it is a biomarker working for the repair of DNA.

O-GlcNAc modification of histones is measured by fixing the target cells for measurement by a conventional method and visualizing by an immunofluorescence staining method (e.g., immunostaining using anti-histone O-GlcNAc antibody). That is, it can be evaluated using, as an index, a significant increase in the number of signals of O-GlcNAc modification of histones in glucose-sensitive cells under high glucose concentration conditions with reaction of the agent of the present invention, relative to the signals of O-GlcNAc modification of histones in glucose-sensitive cells under high glucose concentration conditions without reaction of the agent of the present invention.

In the present invention, "protection from nerve cell disappearance" means suppression of disappearance of nerve cells exposed to high glucose concentration conditions, or recovery from disappearance of nerve cells. The protection from nerve cell disappearance can be evaluated by using, as an index, the absence of significant decrease in the number of nerve cells under high glucose concentration conditions as compared to the number of nerve cells under low glucose concentration conditions, or a significant increase in the number of nerve cells under high glucose concentration conditions with reaction of the agent of the present invention, relative to the number of nerve cells under high glucose concentration conditions without reaction of the agent of the present invention. As used herein, the high glucose concentration refers to a glucose concentration in a medium or body fluid (preferably, blood) of not less than 7.1 mM (preferably, 25 mM), and the low glucose concentration refers to a glucose concentration in a medium or body fluid (preferably, blood) of less than 7.1 mM (preferably, 5 - 7 mM).

In the present invention, N-acetyl-D-mannosamine may be an N-acetyl form of D-mannosamine, which is represented by the following formula (I):

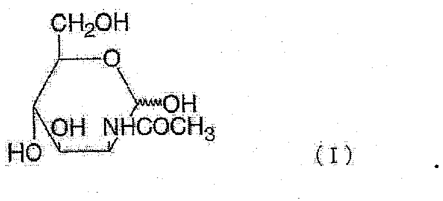

A compound represented by the formula (I) is sometimes to be abbreviated as "ManNAc".

In the present invention, N-acetyl-D-mannosamine is not limited to the compound represented by the above-mentioned formula (I), and is a concept including a derivative, precursor or prodrug thereof, a salt thereof, a solvate thereof (hereinafter sometimes to be abbreviated as "derivative etc.").

N-acetyl-D-mannosamine may be, for example, a compound represented by the following formula (II): 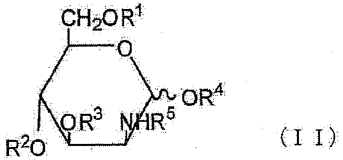 wherein R¹, R², R³ and R⁴ are each independently a hydrogen atom, R⁶, -C(=O)R⁶, -C(=O)OR⁶, or -C(=O)NR⁶R⁷, R⁶ is C₁₋₇ chain hydrocarbon or cyclic hydrocarbon optionally having substituent(s), and R⁷ is a hydrogen atom, or C₁₋₇ chain hydrocarbon or cyclic hydrocarbon optionally having substituent(s).

R⁵ is a hydrogen atom, R⁶, -C(=O)R⁶, -C(=O)OR⁶, -C(=O)NR⁶R⁷, or -C(=O)-CH₂-R⁸, R⁶ is C₁₋₇ chain hydrocarbon or cyclic hydrocarbon optionally having substituent(s), R⁷ is a hydrogen atom or C₁₋₇ chain hydrocarbon or cyclic hydrocarbon optionally having substituent(s), R⁸ is a hydrogen atom or C₁₋₇ chain hydrocarbon or cyclic hydrocarbon optionally having substituent (s), -(CH₂)ₙ-C(=O)R⁹ (n is an integer of 1 - 6, and R⁹ is C₁₋₆ alkyl), -NH-C(=O)R¹⁰ (R¹⁰ is C₁₋₇ chain hydrocarbon optionally having substituent(s)), azido, oxycarbonyl-C₁₋₆ alkyl, or thiocarbonyl-C₁₋₆ alkyl.

As the substituent, a halogen atom (fluorine, chlorine, bromine, iodine) can be used.

In a preferable embodiment, a compound represented by the formula (II) is a derivative wherein R¹, R², R³ and R⁴ are each a hydrogen atom, and R⁵ is -C(=O)OCH₃ (to be referred to as ManNCOOMe): 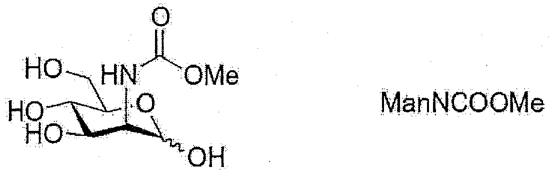 or a derivative wherein R¹, R², R³ and R⁴ are each a hydrogen atom, and R⁵ is -C(=O)OCH₂CH₃ (to be referred to as ManNCOOEt) :

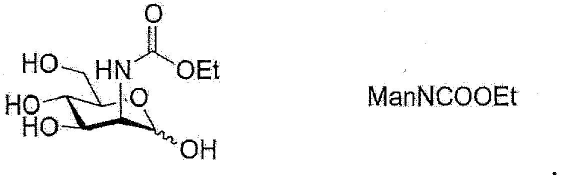

In another embodiment, a compound represented by the formula (II) is preferably the following ManNAcF, ManNAcF₂ or Ac₄ManNAc. 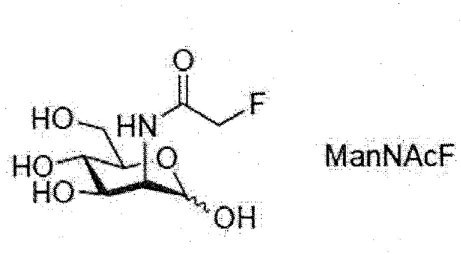 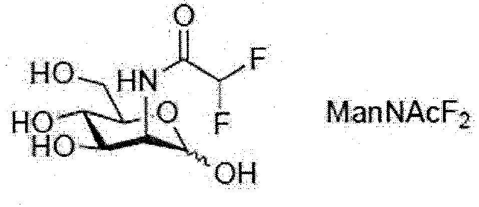 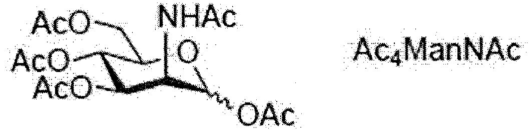 wherein Ac is an acetyl group.

N-acetyl-D-mannosamine may be, for example, a compound represented by the following formula (IIa) - (IIc) and (IIIa) - (IIIc), and further, SPh-αβ-ManNAc.

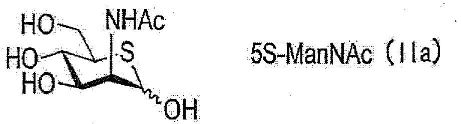

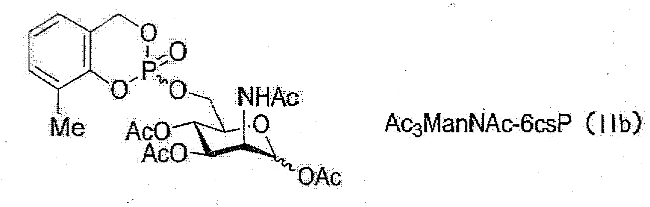

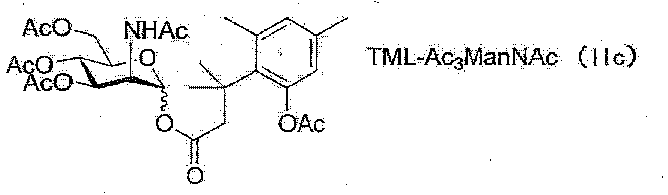

In the above-mentioned formulas, Ac is an acetyl group.

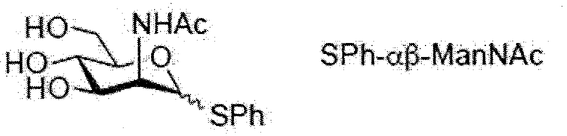

In the above-mentioned formula, SPh is a sphingosine residue (-O-CH₂-CH(NH₂)-CH(OH)-CH=CH-(CH₂)₁₂CH₃), and Ac is an acetyl group.

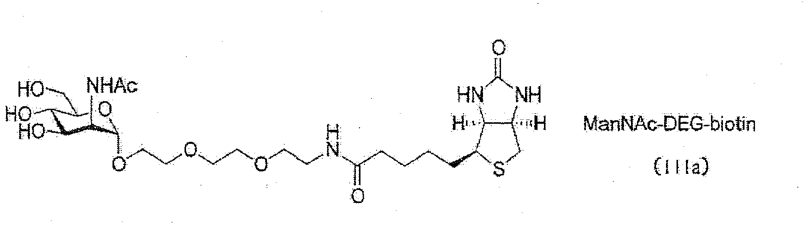

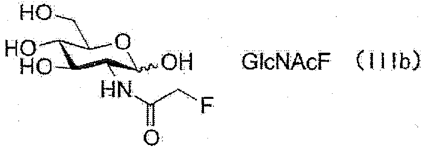

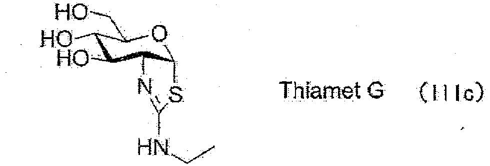

In the above-mentioned formulas, Ac is an acetyl group.

In another preferable embodiment, N-acetyl-D-mannosamine is a compound represented by the following formula (IVa) - (IVc).

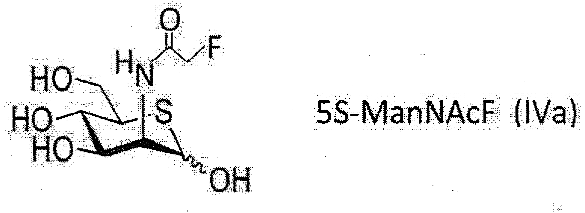

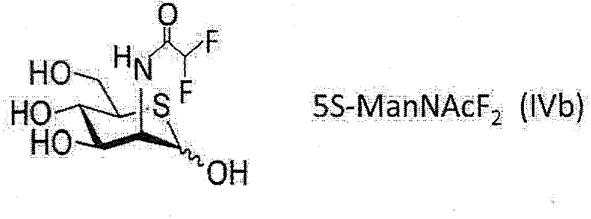

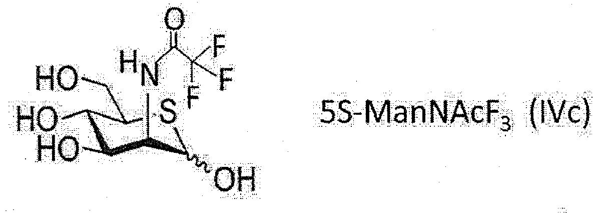

A preferable compound of N-acetyl-D-mannosamine includes ManNAc(I), SPh-αβ-ManNAc, ManNAcF, ManNAcF₂, 5S-ManNAc(IIa), Ac₄ManNAc and Ac₃ManNAc-6csP(IIb).

A more preferable compound of N-acetyl-D-mannosamine includes ManNAc(I), 5S-ManNAc(IIa), 5S-ManNAcF(IVa), 5S-ManNAcF₂(IVb), 5S-ManNAcF₃(IVc) and a salt thereof, furthermore preferably 5S-ManNAcF(IVa).

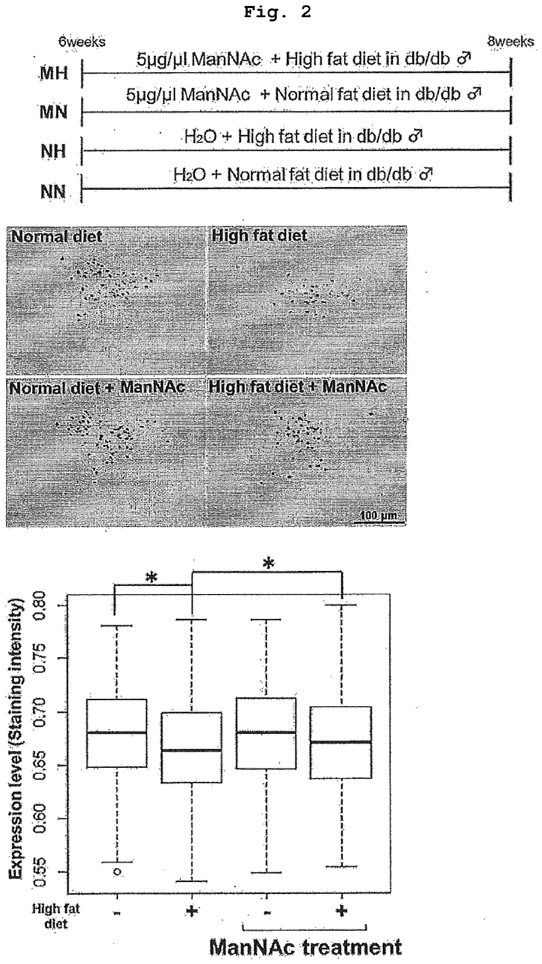

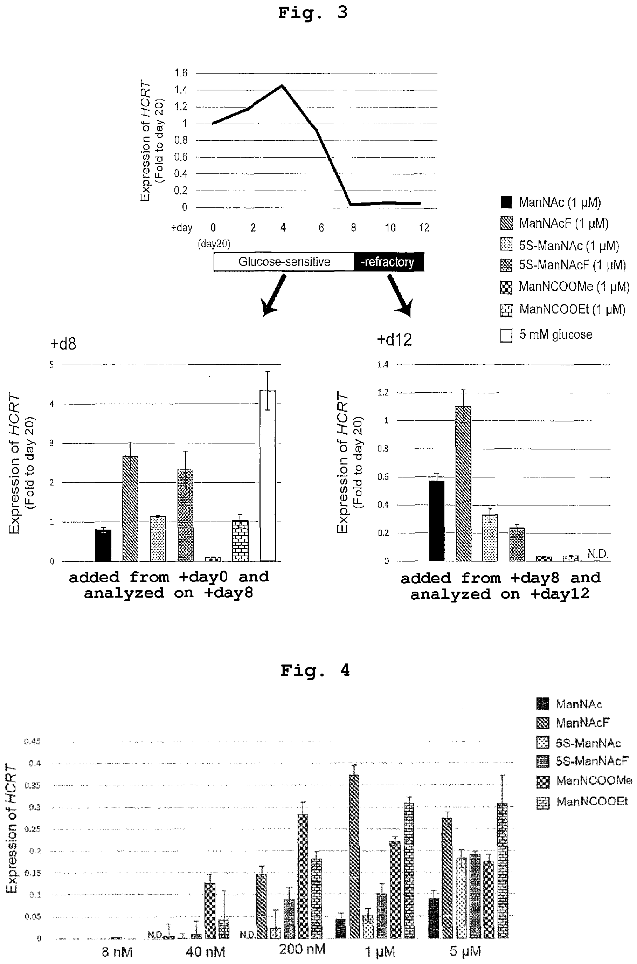

The derivative etc. of N-acetyl-D-mannosamine are also described in documents (Metabolic glycoengineering: Sialic acid and beyond Glycobiology 2009 vol. 19 (12) pp. 1382-1401 (particularly, Fig. 4), Metabolic oligosaccharide engineering with N-Acyl functionalized ManNAc analogs: Cytotoxicity, metabolic flux, and glycan-display considerations Biotechnol Bioeng 2011 vol. 109 (4) pp. 992-1006 (particularly, Fig. 2)), and they can also be used preferably in the present invention.

Examples of the salt of N-acetyl-D-mannosamine include pharmacologically acceptable salts, for example, salts with inorganic acids, salts with organic acids, salts with basic or acidic amino acids and the like.

Examples of the salts with inorganic acids include salts with hydrochloric acid, hydrobromic acid, nitric acid, sulfuric acid, phosphoric acid and the like.

Examples of the salts with organic acids include salts with benzoic acid, formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Examples of the salts with basic amino acids include salts with arginine, lysine, ornithine and the like, and examples of the salts with acidic amino acid include salts with aspartic acid, glutamic acid and the like.

Examples of the solvate preferably include hydrate (e.g., monohydrate, dihydrate and the like), ethanolate and the like.

N-acetyl-D-mannosamine may be a commercially available product, and one produced by a known method may also be used. Examples of the production method of N-acetyl-D-mannosamine represented by the formula (I) include, but are not limited to, a method including isomerization of N-acetylglucosamine under alkaline conditions (JP-A-hei10-182685), and a method including reacting N-acetylneuraminic acid lyase with sialic acid as a substrate (JP-A-2001-78794). The derivative etc. of N-acetyl-D-mannosamine can also be produced by a method known per se by using N-acetyl-D-mannosamine represented by the formula (I) as a starting material.

The compounds represented by the formula (IIa) and the formulas (IVa) - (IVc) are collectively referred to as 5S form in the present invention. Of the 5S forms, a production method of 5S-ManNAc is indicated in Hasegawa. E. Tanahashi, Y. Hioki, M. Kiso, Carbohydrate Res., 122, 168-173 (1983). 5S-ManNAc can be led to 5S-ManNAcF by hydrolysis with hydrochloric acid, followed by treatment with fluoromethyl acetate. 5S-ManNAcF₂ and 5S-ManNAcF₃ can also be synthesized with a similar method.

The agent of the present invention may be N-acetyl-D-mannosamine alone or can be used as a medicament or a food with health claims or a food additive by formulating tablet, pill, granule, fine granule, powder, pellet, capsule, solution, milky lotion, suspension, syrup and pastille and the like containing excipient (e.g., lactose, sucrose, starch, cyclodextrin etc.) and, in some cases, flavor, dye, seasoning, stabilizer, preservative and the like. In addition, the agent of the present invention can also be used as a reagent for research.

While the amount of N-acetyl-D-mannosamine to be contained in the agent of the present invention is not particularly limited as long as the effect of the invention can be afforded, it is generally 0.0001 - 100 wt%, and preferably 0.001 - 99.9 wt%.

When the agent of the present invention is used as a reagent for research, N-acetyl-D-mannosamine can be added to a content of 1 µM - 1 mM in the medium.

The present invention also provides a pharmaceutical composition for protecting nerve cells from disappearance due to high glucose concentration, which contains an effective amount of N-acetyl-D-mannosamine and a pharmaceutically acceptable carrier, or a pharmaceutical composition for protecting glucose-sensitive cells from glucose toxicity due to high glucose concentration.

Examples of the pharmaceutically acceptable carrier include, but are not limited to, excipient (e.g., lactose, sucrose, dextrin, hydroxypropylcellulose, polyvinylpyrrolidone etc.), disintegrant (e.g., starch, carboxymethylcellulose etc.), lubricant (e.g., magnesium stearate etc.), surfactant (e.g., sodium lauryl sulfate etc.), solvent (e.g., water, brine, soybean oil etc.), preservative (e.g., p-hydroxybenzoic acid ester etc.) and the like.

While the effective amount of N-acetyl-D-mannosamine is not particularly limited as long as it affords an effect as a medicament, it is generally 0.0001 - 99.5 wt%, and preferably 0.001 - 99.0 wt%.

The agent or pharmaceutical composition of the present invention can be administered orally or parenterally to mammals (e.g., mouse, rat, leporine, feline, canine, swine, bovine, equine, simian, human).

The present invention provides a food added with N-acetyl-D-mannosamine as an agent for protecting nerve cells from disappearance due to high glucose concentration, or an agent for protecting glucose-sensitive cells from glucose toxicity due to high glucose concentration.

While the "food" of the present invention means general foods and includes general foods including what is called a health food, as well as food with health claims such as food for specified health uses, food with nutrient function claims and the like specified by the health function food system of the Ministry of Health, Labor and Welfare. Furthermore, supplement, feed and the like are also encompassed in the food of the present invention.

In the case of food use, N-acetyl-D-mannosamine can also be used by, for example, adding to general foods (including what is called health food) such as bread, confectionery and the like. In addition, N-acetyl-D-mannosamine can be used as a food with health claims such as food for specified health uses, food with nutrient function claims and the like, or supplement by formulating it in tablet, pill, granule, fine granule, powder, pellet, capsule, solution, milky lotion, suspension, syrup and pastille and the like together with excipient (e.g., lactose, sucrose, starch etc.) and, in some cases, flavor, dye and the like. The food of the present invention can also be applied to feed use, and can be ingested by or administered to poultry, domestic animals and the like by adding to general feed.

For ingestion as food or feed, ingestion frequency per day and ingestion amount for one time of the food or feed are roughly estimated, daily intake is defined, and the amount of N-acetyl-D-mannosamine to be contained in the daily intake of the food or feed is determined. The content of N-acetyl-D-mannosamine can be determined based on the dose mentioned below.

The intake or dose of the agent, food or pharmaceutical composition of the present invention varies depending on the age, body weight and health condition of the subject of intake or administration, and cannot be determined unconditionally. For example, it is preferable to ingest or take 0.1 - 10 g, preferably 0.2 g - 7 g, of N-acetyl-D-mannosamine in one to several portions per day for an adult.

The administration method of the medicament (agent or pharmaceutical composition) of the present invention is not particularly limited as long as it is a route capable of affording a prophylactic or therapeutic effect for nerve cell disappearance due to high glucose concentration, or protection or recovery of glucose-sensitive cells from glucose toxicity due to high glucose concentration. For example, parenteral administration (intravenous administration, intramuscular administration, direct administration into the tissue, intranasal administration, intradermal administration, administration into the cerebrospinal fluid and the like) or oral administration can be adopted for administration. Particularly, for application of the medicament to human, intravenous, intramuscular or oral administration can be adopted. The dosage form is not particularly limited, and various administration dosage forms, for example, oral preparation (granule, powder, tablet, sublingual tablet, film coating agent, sublingual film preparation, capsule, syrup, emulsion, suspension and the like), injection, drip infusion, external preparation (preparations for nasal administration, dermal preparation, ointment and the like) can be used for administration.

The present invention also provides use of N-acetyl-D-mannosamine in producing a medicament for protecting nerve cells from disappearance due to high glucose concentration, or a medicament for protecting glucose-sensitive cells from glucose toxicity due to high glucose concentration. To be specific, it provides a production method of a medicament for the prophylaxis or treatment of nerve cell disappearance due to high glucose concentration, or a medicament for the protection or recovery of glucose-sensitive cells from glucose toxicity due to high glucose concentration, which uses N-acetyl-D-mannosamine.

A method known per se in the pharmaceutical field can be used without limitation for the production method of the medicament of the present invention.

The medicament of the present invention can be used in combination with one or more other drugs. In this case, the combination of the drugs is safer or more effective than the use of either drug alone. Such other drugs can be administered in the route and amount generally used for the drugs and simultaneously or continuously with the medicament of the present invention. When the medicament of the present invention is simultaneously used with one or more other drugs, a pharmaceutical composition in a unit dosage form containing such other drugs and N-acetyl-D-mannosamine is preferable. The combination therapy also includes a therapy comprising administering N-acetyl-D-mannosamine and one or more other drugs according to different overlapping schedules. When used in combination with one or more other active ingredients, use at a smaller dose of N-acetyl-D-mannosamine and the aforementioned other active ingredients than single use of each of them can also be assumed. Therefore, the pharmaceutical composition of the present invention includes one containing N-acetyl-D-mannosamine and one or more other active ingredients. The aforementioned combination includes not only N-acetyl-D-mannosamine and one other active compound, but also a combination with two or more other active compounds.

The weight ratio of N-acetyl-D-mannosamine to the second active ingredient may vary, and depends on the effective dose of each component. In general, each effective dose is used. Therefore, for example, when N-acetyl-D-mannosamine is combined with other drug, the weight ratio of N-acetyl-D-mannosamine to other drug is generally about 1000:1 to about 1:1000, preferably about 200:1 to about 1:200. The combination of N-acetyl-D-mannosamine with other active ingredient is also generally within the aforementioned range. In each case, an effective dose of each active ingredient should be used. In such combination, N-acetyl-D-mannosamine and other active drug(s) may be administered individually or together. Furthermore, administration of one factor may be before administration of another drug, simultaneously with the administration, or after the administration.

The medicament of the present invention can be used in combination with a drug currently used for the treatment of diabetes or a drug currently under development as a therapeutic agent for diabetes. The medicament of the present invention can be administered in combination with other compounds known in the art to be useful for treating diabetes. The combined use of the medicament of the present invention and these therapeutic agents is useful for not only diabetes but also complications associated with diabetes such as diabetic neuropathy, diabetic retinopathy, diabetic nephropathy, vascular complication, skin complication, immunodeficiency and the like.

Examples of the therapeutic agent for diabetes that can be used in combination with the medicament of the present invention include the following medicaments.

Insulin preparation (e.g., animal insulin preparation extracted from pancreas of bovine, swine; human insulin preparation synthesized using Escherichia coli or yeast by genetic engineering; insulin zinc; protamine insulin zinc; fragment or derivative of insulin (e.g., INS-1 etc.), oral insulin preparation etc.), insulin sensitizer (e.g., pioglitazone or a salt thereof (preferably, hydrochloride), rosiglitazone or a salt thereof (preferably, maleate), metaglidasen, AMG-131, Balaglitazone, MBX-2044, Rivoglitazone, Aleglitazar, Chiglitazar, Lobeglitazon, PLX-204, PN-2034, GFT-505, THR-0921, compounds described in WO2007/013694, WO2007/018314, WO2008/093639 or WO2008/099794, etc.), α-glucosidase inhibitor (e.g., voglibose, acarbose, miglitol, emiglitate etc.), biguanide (e.g., metformin, buformin or a salt thereof (e.g., hydrochloride, fumarate, succinate etc.) etc.), insulin secretagogue (e.g., sulfonylurea (e.g., tolbutamide, glibenclamide, gliclazide, chlorpropamide, tolazamide, acetohexamide, glyclopyramide, glimepiride, glipizide, glybuzole etc.), repaglinide, nateglinide, mitiglinide or calcium salt hydrate thereof etc.), dipeptidyl peptidase IV inhibitor (e.g., Alogliptin or a salt thereof (preferably, benzoate), Vildagliptin, Sitagliptin, Saxagliptin, BI1356, GRC8200, MP-513, PF-00734200, PHX1149, SK-0403, ALS2-0426, TA-6666, TS-021, KRP-104, 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof etc.), β3 agonist (e.g., N-5984 etc.), GPR40 agonist (e.g., compounds described in WO2004/041266, WO2004/106276, WO2005/063729, WO2005/063725, WO2005/087710, WO2005/095338, WO2007/013689 or WO2008/001931 etc.), GLP-1 receptor agonist (e.g., GLP-1, GLP-1 MR agent, Liraglutide, Exenatide, AVE-0010, BIM-51077, Aib(8,35)hGLP-1(7,37)NH2, CJC-1131, Albiglutide etc.), amylin agonist (e.g., pramlintide etc.), phosphotyrosine phosphatase inhibitor (e.g., sodium vanadate etc.), gluconeogenesis inhibitor (e.g., glycogen phosphorylase inhibitor, glucose-6-phosphatase inhibitor, glucagon antagonist, FBPase inhibitor etc.), SGLT2 (sodium-glucose cotransporter 2) inhibitor (e.g., Depagliflozin, AVE2268, TS-033, YM543, TA-7284, Remogliflozin, ASP1941 etc.), SGLT1 inhibitor, 11β-hydroxysteroid dehydrogenase inhibitor (e.g., BVT-3498, INCB-13739 etc.), adiponectin or agonist thereof, IKK inhibitor (e.g., AS-2868 etc.), leptin resistance improving drug, somatostatin receptor agonist, glucokinase activator (e.g., Piragliatin, AZD1656, AZD6370, TTP-355, compounds described in WO2006/112549, WO2007/028135, WO2008/047821, WO2008/050821, WO2008/136428 or WO2008/156757 etc.), GIP (Glucose-dependent insulinotropic peptide), GPR119 agonist (e.g., PSN821, MBX-2982, APD597 etc.), FGF21, FGF analogue and the like.

Examples of the therapeutic agent for diabetic complications that can be used in combination with the medicament of the present invention include aldose reductase inhibitor (e.g., tolrestat, epalrestat, zopolrestat, fidarestat, CT-112, Ranirestat (AS-3201), Lidorestat etc.), neurotrophic factor and increasing drugs thereof (e.g., NGF, NT-3, BDNF, neurotrophin producing agent/secretagogue described in WO01/14372(e.g., 4-(4-chlorophenyl)-2-(2-methyl-1-imidazolyl)-5-[3-(2-methylphenoxy)propyl]oxazole etc.), compounds described in WO2004/039365 etc.), PKC inhibitor (e.g., ruboxistaurin mesylate etc.), AGE inhibitor (e.g., ALT946, N-phenacylthiazolium bromide (ALT766), EXO-226, Pyridorin, pyridoxamine etc.), GABA receptor agonist (e.g., gabapentin, Pregabalin etc.), serotonin-noradrenaline reuptake inhibitor (e.g., duloxetine etc.), sodium channel inhibitor (e.g., Lacosamide etc.), active oxygen scavenger (e.g., thioctic acid etc.), cerebral vasodilator (e.g., tiapuride, mexiletine etc.), somatostatin receptor agonist (e.g., BIM23190 etc.), apoptosis signal regulating kinase-1 (ASK-1) inhibitor and the like.

Examples of the therapeutic agent for hyperlipidemia that can be used in combination with the medicament of the present invention include HMG-CoA reductase inhibitor (e.g., pravastatin, simvastatin, lovastatin, atorvastatin, fluvastatin, rosuvastatin, pitavastatin or a salt thereof (e.g., sodium salt, calcium salt etc.) etc.), squalene synthase inhibitor (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), fibrate compound (e.g., bezafibrate, clofibrate, simfibrate, clinofibrate etc.), anion exchange resin (e.g., colestyramine etc.), probucol, nicotinic acid drug (e.g., nicomol, niceritrol, niaspan etc.), ethyl icosapentate, phytosterol (e.g., soysterol, gamma oryzanol (γ-oryzanol) etc.), cholesterol absorption inhibitor (e.g., Zetia etc.), CETP inhibitor (e.g., dalcetrapib, anacetrapib etc.), ω-3 fatty acid preparation (e.g., ω-3-acid ethyl esters 90 etc.) and the like.

Examples of the antiobestic agent that can be used in combination with the medicament of the present invention include monoamine uptake inhibitor (e.g., phentermine, sibutramine, mazindol, fluoxetine, tesofensine etc.), serotonin 2C receptor agonist (e.g., Lorcaserin etc.), serotonin 6 receptor antagonist, histamine H3 receptor modulating drug, GABA modulating drug (e.g., topiramate etc.), neuropeptide Y antagonist (e.g., velneperit etc.), cannabinoid receptor antagonist (e.g., rimonabant, Taranabant etc.), ghrelin antagonist, ghrelin receptor antagonist, ghrelin acylation enzyme inhibitor, opioid receptor antagonist (e.g., GSK-1521498 etc.), melanocortin 4 receptor agonist, 11β-hydroxysteroid dehydrogenase inhibitor (e.g., AZD-4017 etc.), pancreatic lipase inhibitor (e.g., orlistat, cetilistat etc.), β3 agonist (e.g., N-5984 etc.), diacylglycerol acyltransferase 1 (DGAT1) inhibitor, acetyl CoA carboxyrase (ACC) inhibitor, stearic acid CoA desaturase inhibitor, microsomal triglyceride transfer protein inhibitor (e.g., R-256918 etc.), Na-glucose cotransportor inhibitor (e.g., JNJ-28431754, remogliflozin etc.), NFκB inhibitor (e.g., HE-3286 etc.), PPAR agonist (e.g., GFT-505, DRF-11605 etc.), phosphotyrosine phosphatase inhibitor (e.g., sodium vanadate, Trodusquemin etc.), GPR119 agonist (e.g., PSN-821 etc.), glucokinase activator (e.g., AZD-1656 etc.), leptin, leptin derivative (e.g., metreleptin etc.), CNTF (ciliary neurotrophic factor), BDNF (brain derived from neurotrophic factor), cholecystokinin agonist, glucagon-like peptide-1 (GLP-1) preparation (e.g., animal GLP-1 preparation extracted from pancreas of bovine, swine; human GLP-1 preparation synthesized using Escherichia coli or yeast by genetic engineering; fragment or derivative of GLP-1 (e.g., exenatide, liraglutide etc.) etc.), amylin preparation (e.g., pramlintide, AC-2307 etc.), neuropeptide Y agonist (e.g., derivative of PYY3-36, PYY3-36, obinepitide, TM-30339, TM-30335 etc.), oxyntomodulin preparation: FGF21 preparation (e.g., animal FGF21 preparation extracted from pancreas of bovine, swine; human FGF21 preparation synthesized using Escherichia coli or yeast by genetic engineering; fragment or derivative of FGF21 etc.), anorexigenic agent (e.g., P-57 etc.) and the like.

Furthermore, glycosylation inhibitor (e.g., ALT-711 etc.), nerve regeneration promoting drug (e.g., Y-128, VX853, prosaptide etc.), antidepressant (e.g., desipramine, amitriptyline, imipramine etc.), antiepileptic (e.g., lamotrigine, Trileptal, Keppra, Zonegran, Pregabalin, Harkoseride, carbamazepine etc.), antiarrhythmic drug (e.g., mexiletine etc.), acetylcholine receptor ligand (e.g., ABT-594 etc.), endothelin receptor antagonist (e.g., ABT-627 etc.), monoamine uptake inhibitor (e.g., tramadol etc.), narcotic analgesic (e.g., morphine etc.), GABA receptor agonist (e.g., gabapentin, gabapentin MR agent etc.), α2 receptor agonist (e.g., clonidine etc.), topical analgesic (e.g., capsaicin etc.), antianxiety drug (e.g., benzothiazepine etc.), phosphodiesterase inhibitor (e.g., sildenafil etc.), dopamine receptor agonist (e.g., apomorphine etc.), midazolam, ketoconazole and the like can also be used in combination with the medicament of the present invention.

The timing of administration of the medicament of the present invention and the combination drug is not limited, and they may be administered simultaneously or in a staggered manner to a subject.

The administration form is not particularly limited, and the medicament of the present invention and a combination drug only need to be combined. Examples of such administration form include
(1) administration of a single preparation obtained by simultaneously processing the medicament of the present invention and the combination drug,
(2) simultaneous administration of two kinds of preparations of the medicament of the present invention and the combination drug, which have been separately produced, by the same administration route,
(3) administration of two kinds of preparations of the medicament of the present invention and the combination drug, which have been separately produced, by the same administration route in a staggered manner,
(4) simultaneous administration of two kinds of preparations of the medicament of the present invention and the combination drug, which have been separately produced, by different administration routes,
(5) administration of two kinds of preparations of the concomitant of the present invention and the combination drug, which have been separately produced, by different administration routes in a staggered manner (for example, administration in the order of the medicament of the present invention and the combination drug, or in the reverse order) and the like.

The dose of the combination drug can be appropriately determined based on the dose employed clinically. In addition, the mixing ratio of the medicament of the present invention and the combination drug can be appropriately determined according to a subject, administration route, target disease, condition, combination, and the like. For example, when the subject is a human, the combination drug may be used in an amount of 0.01 to 100 parts by weight per 1 part by weight of the medicament of the present invention.

By combining the medicament of the present invention and a combination drug, superior effects such as:
(1) the dose of the medicament of the present invention or a combination drug can be reduced as compared to single administration of the medicament of the present invention or a combination drug,
(2) a sustained treatment effect can be designed by selecting a combination drug having different action mechanism from that of the medicament of the present invention,
(3) a synergistic effect can be afforded by a combined use of the medicament of the present invention and a combination drug, and the like, can be achieved.

### [Examples]

The present invention is explained more specifically in the following by referring to Examples. The following shows representative Examples which are not limitative, and various applications are possible as long as they do not deviate from the technical idea of the present invention.

### Reference Example 1: Induction of expression of orexin gene (Hcrt) using neuronal differentiation induction system from human iPS cells (preparation of orexin nerve cells) Analysis using stromal cell-derived inducing activity (SDIA) differentiation culture system

Basically, orexin nerve cells differentiated from human iPS cells according to the method described in WO 2013/04773 were used. The conditions of the differentiation culture are as described below.

### Differentiation culture

Human iPS cell line 201B7 (HSP0001) was obtained from BioResource Center, RIKEN, Japan via National BioResource project (MEXT, Japan). Human iPS cells were maintained on a feeder layer of mitomycin C-treated STO/Neo resistant/LIF(SNL) feeder cells in primates ES medium (ReproCELL) supplemented with 5 ng/mL recombinant human bFGF (Wako). For differentiation induction into nerve by SDIA and BMP4 system, human iPS cells were cocultured with PA6 for 20 days. 5 nM BMP4 was supplemented from day 7. From the start of the differentiation culture (day 0), ManNAc was added to a neuronal differentiation medium to 1.0 mM, and the medium was exchanged on days 7, 10 and 14 of differentiation culture. In addition, 5 nM recombinant human BMP4 (Wako) was added from day 7 of the differentiation culture. On day 14 of the differentiation culture, EX-527 (SIGMA-ALDRICH) was added to neuronal differentiation medium to 50 nM, or BADGP (SIGMA-ALDRICH) was added to neuronal differentiation medium to 5 mM, and the cells were harvested on day 20 of the differentiation culture and subjected to RT-PCR.

### Expression analysis by RT-PCR method

Total RNA was extracted from the harvested cells by using RNeasy plus Mini Kit (QIAGEN). Then, using SuperScript III First Strand Synthesis System (Invitrogen), a reverse transcription reaction was performed in a reaction mixture containing total RNA (1 µg) and Oligo(dT), whereby cDNA was synthesized.

PCR reaction was performed on a 10 µl scale, 1 U LA-Taq DNA Polymerase (Takara) was added to 0.5 µl of cDNA, 5 µl of 2xGC Buffer I, 200 µM of each dNTP, 1.5 mM MgCl₂ and final concentration 0.2 µM of each primer and, after heat denaturation at 95°C for 3 min, and PCR was performed under the conditions of (95°C 30 sec, 55°C 30 sec, 72°C 30 sec)x35 cycles (20 cycles for Actb). The primers used are shown below. Hcrt Forward; 5'- CTCCAGGCACCATGAACTTT -3' (SEQ ID NO: 1) Hcrt Reverse; 5'- AGTTCGTAGAGACGGCAGGA -3' (SEQ ID NO: 2) Actb Forward; 5'- TTCTACAATGAGCTGCGTGTGG -3' (SEQ ID NO: 3) Actb Reverse; 5'- ATGGCTGGGGTGTTGAAGGT -3' (SEQ ID NO: 4)

Each PCR product was electrophoresed on 2% agarose gel and stained with ethidium bromide, and the bands were observed by UV irradiation.

### Results

As a result of the expression analysis of the orexin gene (Hcrt) of the harvested cells, expression of Hcrt was observed. The cells obtained in Reference Example 1 were used as orexin nerve cells and subjected to the following experiments.

### Example 1: Disappearance of orexin nerve cells under high glucose concentration and protection or recovery from disappearance

The orexin nerve cells obtained in Reference Example 1 were subjected to the following experiment.

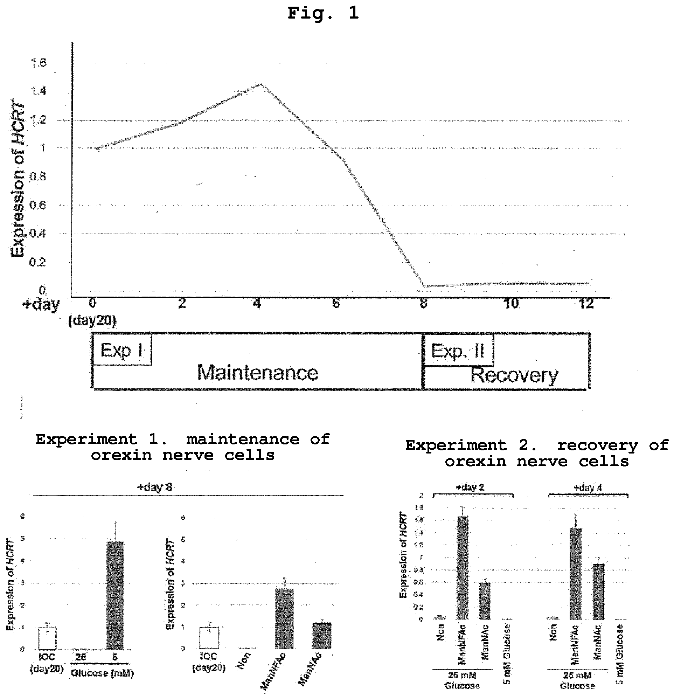

The orexin nerve cells were cultured in a neuronal differentiation medium (G-MEM + 10% KSR (KnockOut Serum Replacement, Invitrogen)) for 8 days, and maintained in vitro. In this case, the cells were divided based on the glucose concentration in the medium into two groups having low glucose concentration (5 mM) and high glucose concentration (25 mM) and cultured. The results are shown in Fig. 1.

When the orexin nerve cells were maintained at high glucose concentration (25 mM), the expression of orexin (Hcrt) fell below the measurable limit and, substantially the orexin nerve cells disappeared (Fig. 1, upper figure). In contrast, when the orexin nerve cells were maintained at low glucose concentration (5 mM), such decrease in the orexin expression or disappearance of orexin cells did not occur (Fig. 1, lower left figure). Therefore, it was clarified that the orexin nerve cells are sensitive to glucose, and orexin nerve cells cannot be maintained under a high concentration glucose environment (Fig. 1, lower left figure).

On the other hand, even under a high glucose concentration, when the orexin nerve cells were once placed back under a low concentration glucose environment, or ManNAc or ManNAcF (also referred to as fluoroManNAc or ManNFAc) which is a derivative thereof was added (addition concentration: 10 µM ManNAc, 1 µM ManNAcF), disappearance of the orexin nerve cells could be avoided (Fig. 1, lower middle figure). Therefore, it was clarified that ManNAc and a derivative thereof have a function to protect nerve cells from neurotoxicity due to high glucose, and are effective for maintaining orexin nerves.

When the number of culture days under a high glucose concentration was extended for 2 or 4 more days, recovery of orexin nerve cells were not seen even when the cells were cultured again under a low concentration glucose environment (Fig. 1, lower right figure). However, when ManNAc and a derivative thereof (ManNAcF) were added to the medium, expression of orexin was observed, thus indicating recovery of the orexin nerve cells (Fig. 1, lower right figure). Therefore, ManNAc and a derivative thereof have an action to recover orexin nerve cells.

On the other hand, since addition of compounds (sirtuin inhibitor EX-527 and OGA inhibitor BADGP) having an action to promote differentiation of orexin cells from undifferentiated cells was completely ineffective (data not shown), ManNAc and a derivative thereof are considered to be important lead compounds capable of recovering nerve cells from the damage due to glucose toxicity (Fig. 1, lower right figure).

### Example 2: Protection of nerve cells from disappearance by ManNAc at individual level

To confirm effectiveness of ManNAc for nerve cell protection at an individual level, the number of orexin nerve cells and orexin expression were examined using genetically obese mouse (db/db mouse, hetero mouse as control, all male) (each group n=6). The breeding conditions of each group (6-8 weeks old) are as described below.
high fat feed+drinking water group
high fat feed+5 µg/µl ManNAc containing drinking water group normal food+drinking water group
normal food+5 µg/µl ManNAc containing drinking water group

### <Detection of orexin gene expression by in situ hybridization method>

Three individual mice from each group mentioned above were subjected to Nembutal anesthesia, and perfusion was fixed with formamide fixative (Genostaff). The brain was taken out, embedded in paraffin, and sections with 6 µm thickness in the sagittal direction were prepared. The sections were stained, for each 10 slides, with hematoxylin-eosin, the position of the section was specified according to the mouse brain atlas (Paxinos G, Franklin KBJ. The mouse brain in stereotaxic coordinates. 2nd edition, Academic Press, 2001), and the section containing a lateral hypothalamic area was subjected to an in situ hybridization method. Sections were deparaffinized with xylene and then stepwisely rehydrated with ethanol and PBS. The sections were fixed with 4% para-formaldehyde/PBS for 15 min, washed with PBS, then treated with 8 g/ml Proteinase K/PBS at 37°C for 30 min, washed with PBS, re-fixed with 4% para-formaldehyde/PBS for 15 min, washed with PBS, treated with 0.2N HCl for 10 min, and washed with PBS. The sections were treated with 0.1 M triethanol amine hydrochloride/0.25 % acetic anhydride for 10 min, and washed with PBS. The sections were stepwisely rehydrated with ethanol and RNA probes diluted to 300 ng/ml with Probe Diluent (Genostaff) were hybridized at 60°C for 16 hr. As the probe, a partial sequence of orexin gene: was cloned into pGEM-1 Easy vector (Promega), and using DIG RNA Labeling Mix (Roche) and the plasmid as a template, digoxigenin-labeled RNA probe was prepared. After hybridization, the sections were each washed with 5×HybriWash (Genostaff) at 60°C for 20 min. Then, RNase treatment was performed in 50 µg/ml RNase A, 10 mM Tris-HCl (pH 8.0), 1 M NaCl, 1 mM EDTA solution at 37°C for 30 min. The sections were washed twice with 2×HybriWash at 60°C for 20 min, twice with 0.2×HybriWash at 60°C for 20 min, and once with TBST (0.1% Tween20/TBS). After blocking treatment with 0.5% blocking reagent (Roche)/TBST for 30 min, the sections were incubated in anti-DIG AP conjugate (Roche) 1000-fold diluted with TBST at room temperature for 2 hr. After washing twice with TBST, they were incubated in 100 mM NaCl, 50 mM MgCl₂, 0.1% Tween20, 100 mM Tris-HCl (pH9.5) solution. Chromogenic reaction was performed overnight using NBT/BCIP (Sigma), and the sections were washed with PBS. The stained sections were counterstained with Kernechtrot (MUTO PURE CHEMICALS), and mounted in Marinol (MUTO PURE CHEMICALS). The cells expressing orexin gene were counted, and the mean of 3 individuals and standard error were determined.

### <Experimental results>

The results are shown in Fig. 2. The number of orexin nerve cells and the orexin gene expression decreased in the group given high fat feed for 6 to 8 weeks of age, as compared to the group raised on normal feed. In contrast, a decrease in the number of orexin nerve cells and the orexin gene expression was suppressed by simultaneously dosing ManNAc even in the group given high fat feed.

### Example 3: Disappearance of orexin nerve cell under high glucose concentration and protection or recovery from disappearance by various ManNAc derivatives

The orexin nerve cells obtained in Reference Example 1 were subjected to the following experiment.

The orexin nerve cells were cultured in a neuronal differentiation medium (G-MEM + 10% KSR (KnockOut Serum Replacement, Invitrogen)) for 8 days or 12 days, and maintained in vitro. In this case, the glucose concentration of the medium was set to a high glucose concentration (25 mM), ManNAc or various ManNAc derivatives (each 1 µM) were added, and the cells were cultured. In the 8-day culture course, glucose and ManNAc or various ManNAc derivatives were added to the medium from the start of the culture (+day0), the cells were harvested on +day8 the orexin expression was analyzed. As a control, orexin nerve cells cultured at low glucose concentration (5 mM) were prepared. In the 12-day culture course, high glucose was added from the start of the culture (+day0), ManNAc or various ManNAc derivatives were added to the medium from +day8 from the start of the culture, the cells were harvested on +day12, and the orexin expression was analyzed. As a control, orexin nerve cells cultured at low glucose concentration (5 mM) from +day8 from the start of the culture were prepared. According to the expression analysis by the RT-PCR method described in Reference Example 1, the expression of Hcrt gene was examined. Based on the expression of Hcrt gene in orexin nerve cells at the start of the culture, expression of Hcrt gene in orexin nerve cells after culture with the addition of ManNAc or various ManNAc derivatives under high glucose conditions is shown by expression intensity (Fig. 3).

Once orexin nerve cells were induced to differentiate and then cultured for more than 8 days in the presence of high glucose, orexin production was not detected (Fig. 3, upper figure). During this period, the production of orexin did not recover even when high glucose was shifted to low glucose (5 mM) (Fig. 3, lower right figure). The derivative ManNAcF showed the highest activity during this period. Other derivatives (5S-ManNAc, 5S-ManNAcF, ManNCOOMe, ManNCOOEt) showed low activity, and the activity of natural form ManNAc was higher (Fig. 3, lower right figure). When differentiation-induced orexin nerve cells were cultured in the presence of high glucose for not more than 8 days, orexin production was maintained except for ManNCOOMe (Fig. 3, lower left figure). In the period of glucose sensitivity, orexin production was also prominent even when cultured under low glucose culture conditions (5 mM) (Fig. 3, lower left figure).

### Reference Example 2: Orexin cell inducibility of ManNAc and derivative thereof

According to the method described in Reference Example 1 except that ManNAc, ManNAcF, 5S-ManNAc, 5S-ManNAcF, ManNCOOMe or ManNCOOEt (8, 40, 200 nM, 1 µM and 5 µM, respectively) was added instead of 1.0 mM ManNAc, orexin cells were induced from human iPS cells. The expression of orexin gene (Hcrt) in the cells induced under the various conditions was examined. The results are shown in Fig. 4.

ManNCOOMe, ManNCOOEt and ManNAcF showed higher orexin cell induction activity as compared to ManNAc. It was shown that the type of highly active ManNAc derivative is different between maintenance (protection or recovery) activity of the orexin cells and the activity of orexin cell induction from iPS cells. It is expected that the action mechanism is different between the maintenance (protection or recovery) activity of the orexin cells and the orexin cell induction activity.

### Example 4: Histone O-GlcNAc modification in renal proximal tubule epithelial cells derived from type II diabetes patients (D-RPTEC)

### Culture of human kidney renal proximal tubule epithelial (RPTEC) cells

Normal human renal proximal tubule epithelial cells and RPTEC derived from type II diabetes patients (D-RPTEC) were purchased from Lonza. RPTEC and D-RPTEC maintained with REGM™ Renal Epithelial Cell proliferation Medium Bullet Kit (Lonza) were each seeded in a 4-well dish at 1x10⁴ cells per 1 well, and cultured for 4 days in a control medium or high glucose medium (adjusted to final concentration of 25 mM with 2.5 M glucose solution).

### Detection of histone O-GlcNAc signal by immunofluorescence staining method

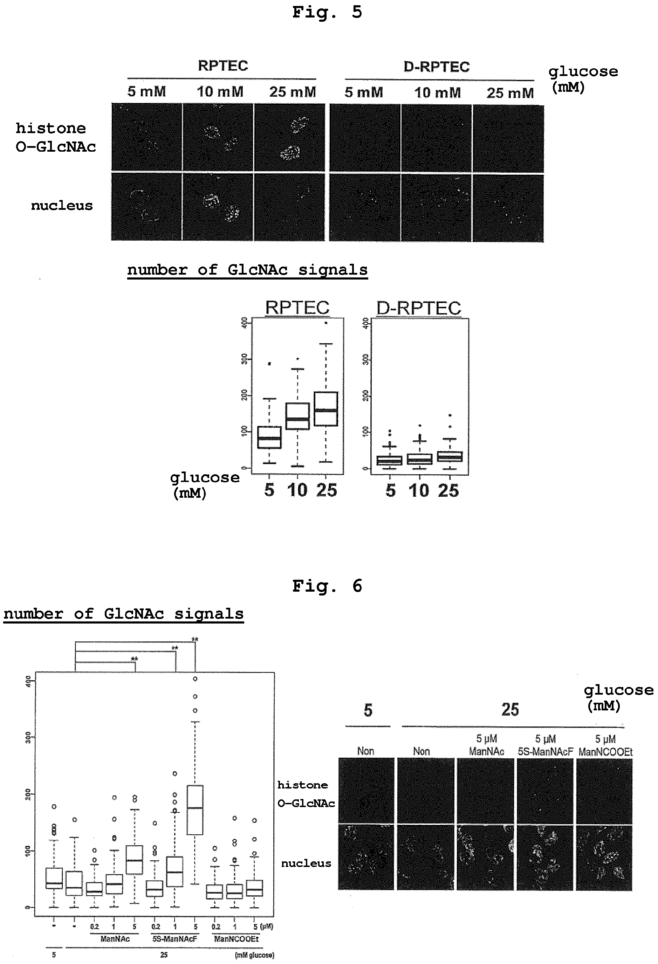

The cultured cells were fixed with 4% para-formaldehyde for 20 min and, after a permeabilization treatment with 0.2% TritonX-100-PBS solution, treated overnight with a blocking solution [PBS(-) containing 5% bovine serum-derived albumin (BSA), 0.1% Tween20, 0.2% TritonX-100] at 4°C. Then, the cells were reacted with anti-histone O-GlcNAc antibody (20B2) diluted with 5% BSA-PBS(-)-0.1% Tween20 at 4°C overnight, reacted with secondary antibody diluted with PBS(-) at room temperature for 1 hr. The nucleus was stained with 1 µg/ml DAPI (Dojindo) at room temperature for 20 min, mounted in mountant (VECTASHIELD), and slides were prepared. Observation was performed with an integrated confocal laser microscope (FV10i, OLYMPUS). After obtaining images, the number of O-GlcNAc signals in the nucleus was measured by image analysis software Cell Profiler (www.cellprofiler.org), and statistical processing was performed by Wilcoxon rank-sum test. The results are shown in Fig. 5.

The analyses heretofore have revealed that the histone O-GlcNAc modification varies in response to extracellular glucose (Japanese patent application No. 2015-177176). In vascular endothelial cells and the like, O-GlcNAc modification increases under high glucose (Japanese patent application No. 2015-177176). Normal cells are considered to have acquired resistance to DNA damage or high repair capacity by increasing histone O-GlcNAc modification in response to glucose.

Increase in histone O-GlcNAc modification under high glucose also in renal proximal tubule epithelial cells (RPTEC); on the other hand, the absence thereof in RPTEC derived from type II diabetes patients (D-RPTEC) (Fig. 5) indicate that the DNA repair capacity acquired in response to glucose is not demonstrated in the cells exhibiting diabetes.

### Example 5: Screening for compounds that vary histone O-GlcNAc modification level

Cells exhibiting diabetes are considered to show low resistance to genomic DNA damage caused by various extracellular stresses. Nevertheless, by utilizing the analysis results of Example 4, namely, by using the histone O-GlcNAc modification level as an index in D-RPTEC, factors that increase DNA repair capacity can be screened for and, results that lead to the treatment of diabetes can be obtained. In this experiment, therefore, ManNAc and two kinds of derivatives thereof (5S-ManNAcF and ManNCOOEt) were added to the medium, the cells were cultured for 96 hr under high glucose (25 mM glucose), and the signals of histone O-GlcNAc were detected in the same manner as in Example 5. The results are shown in Fig. 6.

From Fig. 6, an increase in the histone O-GlcNAc modification level was observed when ManNAc and 5S-ManNAcF were added. That is, these compounds have an action to recover glucose-responsive DNA repair capacity once degraded in D-RPTEC.

### Reference Example 3: Production method of 5S-ManNAcF(IVa)

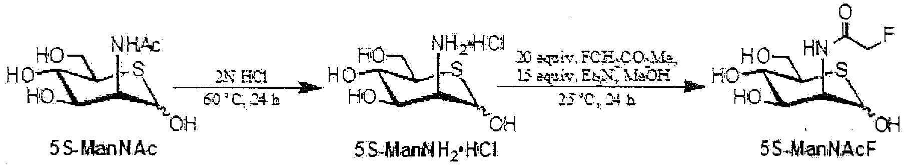

### Synthesis of 2-amino-2-deoxy-5-thio-D-mannopyranose hydrochloride (5-thio-D-mannosamine hydrochloride, 5S-ManNH₂• HCl)

2-acetamido-2-deoxy-5-thio-α-D-mannofuranoside (5S-ManNAc) (100 mg, 0.45 mmol) was dissolved in 2N HCl (1.0 mL) and heated at 60°C for 24 hr. Then, the mixture was concentrated under reduced pressure, and 5S-ManNH₂•HCl (110 mg) was obtained as a pale-brown powder. This compound contained α- and β-anomers at a ratio of 73:27.
¹H NMR (400 MHz, D₂O, ref. HOD at 4.80 ppm at 25°C) α-anomer:δ3.30 (1H, dt, J = 9.4 and 4.5 Hz, 5-H), 3.80 (1H, t, J = 9.4 Hz, 4-H), 3.90 (1H, t, J = 4.0 Hz, 2-H), 3.91 (2H, d, J = 4.5 Hz, 6-H2), 4.09 (1H, dd, J = 9.4 and 4.0 Hz, 3-H),5.17 (1H, d, J = 4.0 Hz, 1-H). β-anomer:δ3.00 (1H, ddd, J = 9.4, 6.3 and 3.6 Hz, 5-H), 3.74 (1H, dd, J = 9.4 and 9.0 Hz, 4-H), 3.83 (1H, dd, J = 11.7 and 6.3 Hz, 6-H),3.84 (1H, dd, J = 9.0 and 4.0 Hz, 3-H),3.96 (1H, dd, J = 11.7 and 3.6 Hz, 6-H), 3.98 (1H, dd, J = 4.0 and 2.7 Hz, 2-H), 5.39 (1H, d, J = 2.7 Hz, 1-H).

### Synthesis of 2-fluoroacetamido-2-deoxy-5-thio-D-mannopyranose (N-fluoroacetyl-5-thio-D-mannosamine, 5S-ManNAcF)

5S-ManNH₂•HCl (110 mg) obtained as mentioned above was dissolved in anhydrous MeOH (3.8 mL), and Et₃N (940 µL, 6.74 mmol) and methylfluoroacetate (700 µL, 8.92 mmol) were successively added at 0°C. The mixture was stirred at 27°C for 24 hr, and concentrated under reduced pressure. The residue was purified by silica gel column chromatography (CHCl₃: MeOH = 2:3), and 5S-ManNAcF (82.2 mg, 76%, yield from 5S-ManNAc) was obtained as a 87:13 mixture of α- and β-anomers.
¹H NMR (500 MHz, D₂O, ref. HOD at 4.80 ppm at 25°C) α-anomer:δ3.30 (1H, ddd, J = 10.0, 5.5 and 3.1 Hz, 5-H), 3.80 (1H, dd, J = 10.0 and 9.7 Hz, 4-H), 3.90 (1H, dd, J = 12.0 and 3.1 Hz, 6-H), 3.98 (1H, dd, J = 12.0 and 5.5 Hz, 6-H), 4.05 (1H, dd, J = 9.7 and 4.1 Hz, 3-H), 4.71 (1H, dd, J = 4.1 and 3.1 Hz, 2-H), 4.95 (1H, d, J = 3.1 Hz, 1-H), 4.98 and 4.99 (each 1H, each dd, each J = 46.1 and 14.2 Hz, -COCH₂F). β-anomer:δ3.03 (1H, ddd, J = 9.6, 6.4 and 3.2 Hz, 5-H), 3.70 (1H, dd, J = 9.6 and 9.3 Hz, 4-H), 3.74 (1H, dd, J = 9.3 and 3.8 Hz, 3-H), 3.91 (1H, dd, J = 12.0 and 6.4 Hz, 6-H), 3.96 (1H, dd, J = 12.0 and 3.2 Hz, 6-H), 4.85 (1H, dd, J = 3.8 and 2.4 Hz, 2-H), 5.03 and 5.04 (each 1H, each dd, each J = 46.8 and 14.4 Hz, -COCH₂F), 5.34 (1H, d, J = 2.4 Hz, 1-H).

### [Industrial Applicability]

The present invention provides a medicament, a food and the like containing N-acetyl-D-mannosamine as an active ingredient. Nerve cell disappear due to glucose toxicity can be prevented or treated and glucose-sensitive cells can be protected or recovered from glucose toxicity by medication or ingestion of the medicament or food of the present invention.

This application is based on a patent application No. 2015-077228 filed in Japan (filing date: April 3, 2015), the contents of which are incorporated in full herein.

## Claims

1. An agent for protecting a glucose-sensitive cell from glucose toxicity due to high glucose concentration, comprising N-acetyl-D-mannosamine.

2. The agent according to claim 1, wherein the glucose-sensitive cell is selected from the group consisting of a nerve cell, a renal cell and a vascular endothelial cell.

3. The agent according to claim 1, wherein the glucose-sensitive cell is selected from the group consisting of a renal cell and a vascular endothelial cell, and protection of the glucose-sensitive cell refers to genome repair in the cell.

4. A pharmaceutical composition for protecting a glucose-sensitive cell from glucose toxicity due to high glucose concentration, comprising an effective amount of N-acetyl-D-mannosamine and a pharmaceutically acceptable carrier.

5. The pharmaceutical composition according to claim 4, wherein the glucose-sensitive cell is selected from the group consisting of a nerve cell, a renal cell and a vascular endothelial cell.

6. The pharmaceutical composition according to claim 4, wherein the glucose-sensitive cell is selected from the group consisting of a renal cell and a vascular endothelial cell, and protection of the glucose-sensitive cell refers to genome repair in the cell.

7. The pharmaceutical composition according to any one of claims 4 to 6, for the treatment of a diabetic complication.

8. Use of N-acetyl-D-mannosamine in producing a medicament for protecting a glucose-sensitive cell from glucose toxicity due to high glucose concentration.

9. The use according to claim 8, wherein the glucose-sensitive cell is selected from the group consisting of a nerve cell, a renal cell and a vascular endothelial cell.

10. The use according to claim 8, wherein the glucose-sensitive cell is selected from the group consisting of a renal cell and a vascular endothelial cell, and protection of the glucose-sensitive cell refers to genome repair in the cell.

11. A method of protecting a glucose-sensitive cell from glucose toxicity due to high glucose concentration, comprising administering an effective amount of N-acetyl-D-mannosamine to a subject in need thereof.

12. The method according to claim 11, wherein the glucose-sensitive cell is selected from the group consisting of a nerve cell, a renal cell and a vascular endothelial cell.

13. The method according to claim 11, wherein the glucose-sensitive cell is selected from the group consisting of a renal cell and a vascular endothelial cell, and protection of the glucose-sensitive cell refers to genome repair in the cell.

14. The method according to any one of claims 11 to 13, for the treatment of a diabetic complication.

15. An agent for protecting a nerve cell from disappearance due to high glucose concentration, comprising N-acetyl-D-mannosamine.

16. The agent according to claim 15, wherein the nerve cell is an orexin nerve cell.

17. A pharmaceutical composition for the prophylaxis or treatment of nerve cell disappearance due to high glucose concentration, comprising an effective amount of N-acetyl-D-mannosamine and a pharmaceutically acceptable carrier.

18. The pharmaceutical composition according to claim 17, wherein the nerve cell is an orexin nerve cell.

19. The pharmaceutical composition according to claim 17 or 18, for the treatment of a diabetic complication.

20. Use of N-acetyl-D-mannosamine in producing a medicament for protecting a nerve cell from disappearance due to high glucose concentration.

21. The use according to claim 20, wherein the nerve cell is an orexin nerve cell.

22. A method for the prophylaxis or treatment of nerve cell disappearance due to high glucose concentration, comprising a step of administering an effective amount of N-acetyl-D-mannosamine to a subject in need thereof.

23. The method according to claim 22, wherein the nerve cell is an orexin nerve cell.

24. The method according to claim 22 or 23, for the prophylaxis or treatment of a diabetic complication.


### SEQUENCE LISTING

<110> The University of Tokyo
<120> Agent for protecting glucose-sensitive cells
<130> 092449
<150> JP 2015-077228
   <151> 2015-04-03
<160> 5
<170> PatentIn version 3.5
<210> 1
   <211> 20
   <212> DNA
   <213> Artificial Sequence
<220>
   <223> Hcrt primer forward
<400> 1
   ctccaggcac catgaacttt 20
<210> 2
   <211> 20
   <212> DNA
   <213> Artificial Sequence
<220>
   <223> Hcrt primer reverse
<400> 2
   agttcgtaga gacggcagga 20
<210> 3
   <211> 22
   <212> DNA
   <213> Artificial Sequence
<220>
   <223> Actb primer forward
<400> 3
   ttctacaatg agctgcgtgt gg 22
<210> 4
   <211> 20
   <212> DNA
   <213> Artificial Sequence
<220>
   <223> Actb primer reverse
<400> 4
   atggctgggg tgttgaaggt 20
<210> 5
   <211> 311
   <212> DNA
   <213> Artificial Sequence
<220>
   <223> Probe
<400> 5
   cgtgttcctg ccgtctctac gaactgttgc acggagctgg caaccacgct gcgggtatcc      60
   tgactctggg aaagcggcgg cctggacctc caggcctcca gggacggctg cagcgcctcc     120
   ttcaggccaa cggtaaccac gcagctggca tcctgaccat gggccgccgc gcaggcgcag     180
   agctagagcc acatccctgc tctggtcgcg gctgtccgac cgtaactacc accgctttag     240
   caccccgggg agggtccgga gtctgaaccc atcttctatc cttgtcctga tccaaacttc     300
   cccctctgct c                                                          311